Europäisches Patentamt

(19) European Patent Office

Office européen des brevets

(11) Publication number: **0 047 270**
**B1**

(12) **EUROPEAN PATENT SPECIFICATION**

(45) Date of publication of patent specification: **03.10.84**

(21) Application number: **81900559.6**

(22) Date of filing: **10.03.81**

(86) International application number:
**PCT/GB81/00038**

(87) International publication number:
**WO 81/02515 17.09.81 Gazette 81/22**

(51) Int. Cl.³: **A 61 F 11/00, A 41 D 21/00, A 45 D 44/12**

(54) **DEVICES FOR PROTECTION OF HUMAN EARS.**

(30) Priority: **13.03.80 GB 8008553**

(43) Date of publication of application:
**17.03.82 Bulletin 82/11**

(45) Publication of the grant of the patent:
**03.10.84 Bulletin 84/40**

(84) Designated Contracting States:
**FR**

(56) References cited:
**US-A-3 112 493**
**US-A-4 134 153**

(73) Proprietor: **OSMAN, Mohammad Fekry.**
**53 Glynderi Tanerdy Carmarthen**
**Dyfed Wales (GB)**

(72) Inventor: **OSMAN, Mohammad Fekry.**
**53 Glynderi Tanerdy Carmarthen**
**Dyfed Wales (GB)**

(74) Representative: **Dunlop, John Henderson et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to devices for protection of human ears. A need for such a device can arise in a number of circumstances. As one example, use of such a device may be desirable during care of a patient following major or minor aural surgery. As another example, such a device may be desirable in the treatment or prevention of external otitis. As yet another example, such a device may be desirable for temporary use in the home, or in a hairdressing salon during application to the hair of material which can be harmful to the ears, e.g. hair sprays, shampoos, lacquers.

Hitherto, in surgical and medical tratment, protection of human ears has usually been provided by the use of bandages.

US Patent 3,112,493 discloses an ear muff, including a dished sheet member with a central generally ellitical opening. In use, the opening receives the wearer's ear, and the sheet member is then snapped from a condition convex towards the head into a condition concave towards the head.

US Patent 4,134,153 discloses a throwaway ear protector made of a square of highly flexible thin film, in the centre of which is a generally D-shaped opening bordered by adhesive.

The present invention relates to devices for use in protection of the human ear, in the form of a self-supporting sheet member, having an opening through the central part of the member to permit the helix of the auricle to pass through the opening, the posterior boundary of the opening being convex rearwards, and the devices are characterised in that the sheet member is substantially flat, and the anterior boundary includes at its mid height a lobe projecting rearwards into the opening, so that the member can be supported on the auricle, with the lobe lying adjacent to the tragus.

In the present description and claims the expression "self-supporting" is used to indicate that a component, in the absence of any external forces, retains a definite shape of its own accord.

In some circumstances, it is sufficient to cover the external auditory meatus, in which case the device can be used alone. In other circumstances the auricle requires to be covered, in which case the device is preferably used in combination with a cover attached, or capable of being attached, to one face of the member, to enclose a space adjacent to that face.

For use, the auricle is passed progressively through the opening in the device until the device is lying with its posterior portion against the head, between the auricle and the head, while the lobe lies adjacent to the tragus. The device can be removed from the ear by a reversal of this manoeuvre, but the size of the opening is such that the device will not become shaken off, nor be accidentally dislodged by slight pressure or impact.

A device according to the invention can be applied to an ear, and removed, by a single person, that is either by a nurse, or by the wearer. In contrast, the application of bandages to protect an ear has hitherto required two or three nurses, in order to support the head in a suitable position, and to apply the bandage at a suitable tension.

A device according to the invention does not impose any constriction on the blood circulation, whereas a bandage tends to constrict the blood supply, and this fact can delay healing.

The use of a device according to the invention facilitates inspection of the condition of an ear, whereas a bandage conceals the ear, and prevents inspection.

A patient wearing a device according to the invention is able to attend to his or her personal appearance, by washing, shaving, applying make-up, brushing hair. This is important for the morale of a patient. In contrast, the presence of a bandage seriously interferes with or prevents all these activities. When used in a hairdressing salon, the provision of such temporary protection has the substantial advantage of reducing the risk that the ears will be harmed in the course of treating the hair.

The accompanying drawings show one example of a device embodying the present invention, in the form of a base plate, together with a cover in the form of a cap. In these drawings:—

Figure 1 is a side elevation of a human left ear;

Figure 2 is a side elevation of the same ear supporting the base plate of the device;

Figure 3 is a rear view, looking in the direction of the arrow III in Figure 2;

Figure 4 is a fragmentary horizontal section looking in the direction of the arrows IV—IV in Figure 2;

Figure 5 is a view of the inner face of the base plate of Figure 2;

Figure 6 is an elevation, looking outwards, of a cap; and

Figure 7 is a vertical section showing the base plate and cap assembled, both components being in section on the lines VII—VII in Figures 5 and 6 respectively.

The device shown in the drawings comprises a base plate 2 intended to be supported on the auricle 4, and a cap intended to be releasably attached to the base plate.

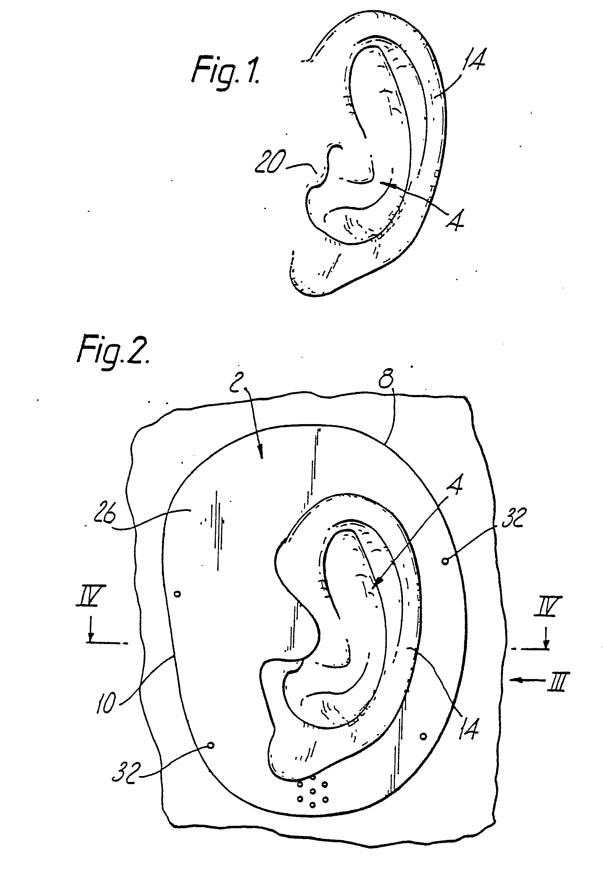

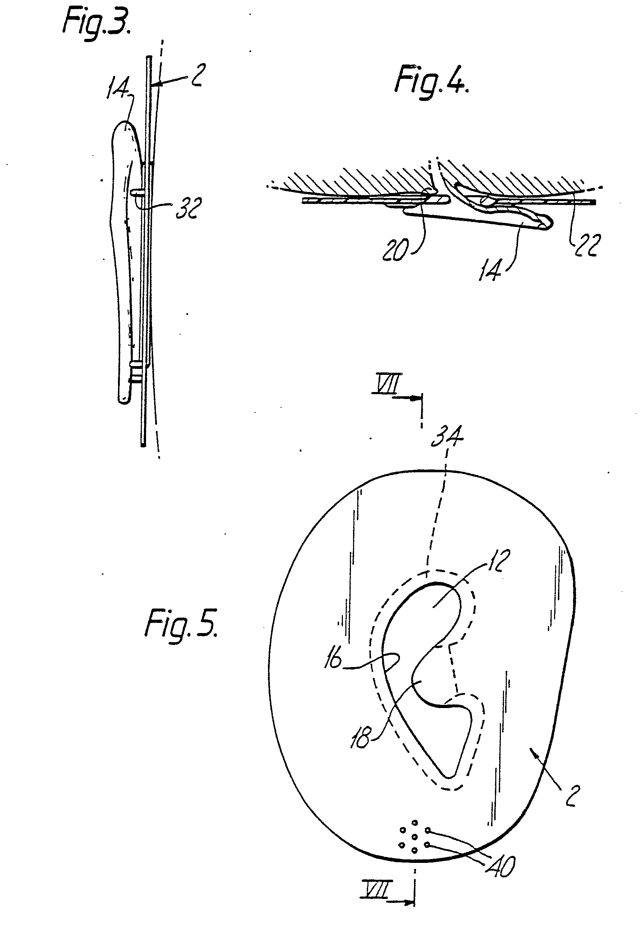

As shown in Figures 2, 3, 4, 5 and 7, the base plate is a substantially flat member, with some local thickening. The majority of the member is of a silicone material which is about 1 mm thick, having properties such that the base plate is self-supporting. As shown in Figure 2, the base plate has an external outline 8 somewhat larger than the outline of the auricle 4. This outline is oval in general nature, with a nearly straight anterior edge portion 10.

Through the central part of the base plate there is an opening 12 of a size to permit the helix 14 of the auricle to pass through the opening. The posterior boundary 16 of the opening is convex rearwards. The anterior boundary includes at its mid-height a lobe 18 projecting rearwards into the opening 12.

As a consequence of possessing the shape described, the base plate can be supported on the auricle, as shown in Figures 2, 3 and 4, with the lobe 18 lying adjacent to the tragus 20 (Figure 1) and with the posterior part of the base plate lying between the auricle 14 and the adjacent surface 22 of the head (Figure 4).

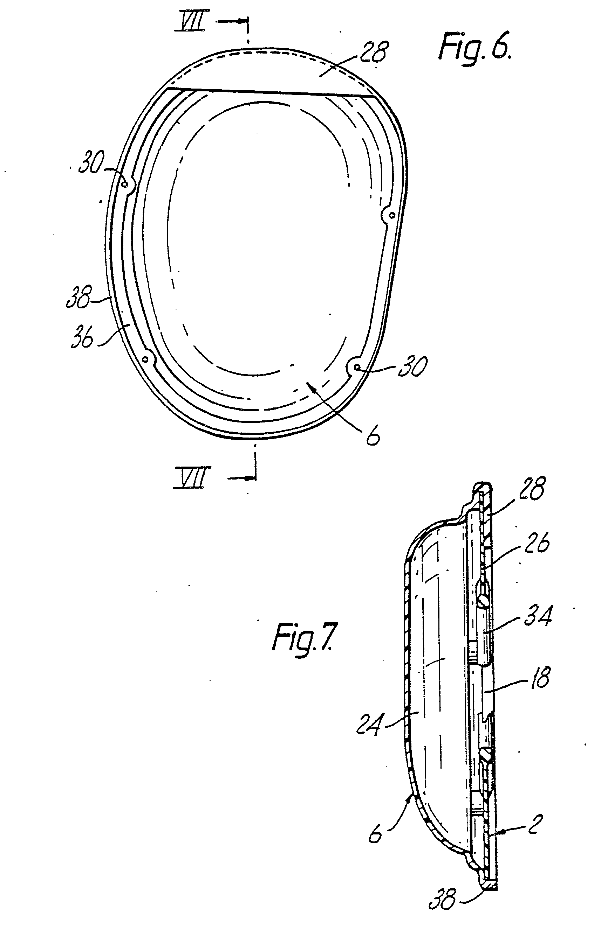

The cap 6 is of a self-supporting plastic-like material, preferably a material which is transparent to at least a substantial extent, e.g. The cap has an outline similar to, and slightly larger than, the base plate, and has a generally concavo-convex shape, as shown in Figure 7. The consequence is that, when the cap is attached to the base plate, it encloses a space 24 adjacent to the face 26 of the base plate, that being the face which, when the base plate is supported on the auricle, is outwards.

The cap is releasably attached to the baseplate by releasable means consisting of a flange 28 extending downwards from an upper portion of the rim of the cap towards a bottom portion of the rim, plus four sockets 30 adjacent to the rim of the cap, which can receive pins 32 (Figures 2 and 3) on the face 26 of the base plate.

To enhance the comfort for a wearer, the opening 12 has a rim 34 which is rounded and is softer than the majority of the base plate. This rim extends around all the boundary of the opening 12, except at the lobe 18.

On the inner face of the lobe 18 (that is to say the face which in use is towards the tragus), the rim of the lobe has a covering of material which is softer than the majority of the base plate.

Preferably the base plate is manufactured by an injection moulding process, and soft material to constitute the rim 34 and the rim of the lobe 18 is prefabricated and placed in the mould cavity prior to injection of the material which constitutes the majority of the base plate.

In order to ensure that liquids or dirt do not readily penetrate into the space 24, the outer periphery of the base plate is received against a shoulder 36 in the cover, and is surrounded by a flange 38 on the cover. Ventilation of the space 24 is provided by ventilation holes 40 between the opening 12 and the lowest portion of the outline of the base plate.

Although the external appearance of the auricle varies considerably from one individual to another, the applicant has found that much of the variety in dimensions is due to variety in size of the lobule at the bottom of the auricle. This lobule is soft, and there is less variation in the dimensions of the more rigid portion of the auricle, it being this more rigid portion which cooperates with the opening 12 in the base plate. The applicant has discovered that the needs of adults and children can be almost entirely met by provision of base plates having a single size of external outline, and four different sizes of opening 12. An indication of the general size of the device can be given by stating that the overall vertical dimension of the base plate as shown in Figures 2 and 5 is in practice about 101 mm.

In post-operative care, it is desirable to use the base plate with the cap attached continuously during the early stages (e.g. the first five days). Thereafter, the cap can be removed, because by then only a small dressing is required at the site of the operation, and the base plate provides adequate protection during normal wear. The cap can be applied temporarily e.g. to enable the wearer to take a shower.

As an alternative to the base plate shown in the drawings, a sheet-like device can be formed from sheet material of uniform thickness. In particular, such devices may be used in conjunction with small dressings of a variety of shapes and in a variety of positions.

Protection is best obtained by providing several alternative sheets from which selection can be made by medical staff.

One sheet has an opening as described above, with posterior and anterior boundaries both C-shaped, convex rearwards. The lobe immediately forward of the opening is preferably permanently bent towards the wearer's head so as to approach the concha when worn.

A second sheet has a lobe which extends further rearwards, so as almost to reach the posterior boundary of the opening. Then out of the lobe is struck a small tongue, directed forwards and towards the wearer's head. In use, this tongue enters the external auditory meatus.

A third sheet has an opening which has a near-vertical anterior edge, so that the opening is roughly D-shaped, and there is a long tongue extending into the opening, upwards and rearwards from the anterior lower corner of the opening. The base of the tongue is narrow, and in use it fits the intertragal area, while the remainder of the tongue is shaped to match the shape of the antihelix of the auricle.

A base plate made by moulding, or a sheet-like device, may be used in conjunction with a cover in the form of a flexible bag. This flexible bag may be permanently attached to the device, round part or all of the periphery of the opening of the bag. The material of the flexible bag should be chosen so that it will not produce unpleasant crackling noises.

The device with a flexible bag may be used in surgical care both before and after the use of a base plate and cap as described above. That is to say, on the night before an operation a patient can be fitted with a device with a bag. This is easier than bandaging to remove in the operating theatre, with less risk of introduction of invection. A device with a bag may also be used in the later stages of post-operative care.

A device with a bag may also be used in the home or in a hairdressing salon, as temporary protection during application to the hair of material which can be harmful to the ears.

**Claims**

1. A device, for use in protection of the human ear, in the form of a self-supporting sheet member (2), having an opening (12) through the central part of the member to permit the helix (14) of the auricle to pass through the opening, the posterior boundary (16) of the opening being convex rearwards, characterised in that the sheet member is substantially flat, and the anterior boundary includes at its mid-height a lobe (18) projecting rearwards into the opening, so that the member can be supported on the auricle, with the lobe lying adjacent to the tragus (20).

2. A device according to claim 1, characterised in that the opening (12) has a rim (34) which is rounded and is softer than the majority of the member (2).

3. A device according to claim 1, characterised in that one face of the lobe (18) is softer than the majority of the member (2).

4. A device according to claim 1, characterised in that the majority of the member (2) is of a silicone material.

5. A device according to claim 1, characterised by combination with a cover (6) attached to, or capable of being attached to, one face of the member (2), to enclose a space (24) adjacent to that face.

6. A device and cover according to claim 5, characterised in that the cover is in the form of a self-supporting cap (6), and there is releasable means (28, 30, 32) for attaching a rim of the cap to the member (2).

7. A device and cover according to claim 6, characterised in that the releasable means consists of a flange (28) extending from a portion of the rim of the cap (6) towards an opposite portion, plus a plurality of pins (32) projecting from the said face of the member (2), and a corresponding plurality of sockets (30) in the cap adjacent to its rim, the pins being a push fit in the sockets.

8. A device and cap according to claim 6, characterised in that there is at least one ventilation hole (40) through the member (2) below the said opening (12).

9. A device and cover according to claim 5, characterised in that the cover is in the form of a flexible bag.

10. A device according to claim 1, characterised in that the member is of sheet material of uniform thickness.

11. A modification of a device according to claim 1, characterised in that in place of the said lobe there is a long tongue extending into the opening, upwards and rearwards from the anterior lower portion of the boundary of the opening, to fit the intertragal area and to match the shape of the antihelix of the auricle.

**Revendications**

1. Dispositif à utiliser pour la protection de l'oreille humaine sous la forme d'un élément autoportant (2) en forme de feuille dont la partie centrale est traversée par une ouverture (12) pour permettre à l'hélix (14) de l'auricule de passer dans cette ouverture, le bord postérieur (16) de l'ouverture étant convexe vers l'arrière, caractérisé en ce que l'élément en forme de feuille est sensiblement plat, et le bord antérieur comporte, à mi-hauteur, un lobe (18) faisant saillie vers l'arrière et vers l'intérieur de l'ouverture, de manière que l'élément puisse être supporté par l'auricule, le lobe s'étendant à proximité immédiate du tragus (20).

2. Dispositif selon la revendication 1, caractérisé en ce que l'ouverture (12) comporte un rebord (34) qui est arrondi et plus mou que la plus grande partie de l'élément (2).

3. Dispositif selon la revendication 1, caractérisé en ce qu'une face du lobe (18) est plus molle que la plus grande partie de l'élément (2).

4. Dispostif selon la revendication 1, caractérisé en ce que la plus grande partie de l'élément (2) est en une matière siliconée.

5. Dispositif selon la revendication 1, caractérisé par la combinaison avec un élément (6) de recouvrement fixé à, ou pouvant être fixé à, une face de l'élément (2), afin de fermer un espace (24) adjacent à cette face.

6. Dispositif et élément de recouvrement selon la revendication 5, caractérisés ce que l'élément de recouvrement se présente sous la forme d'un couvercle autoportant (6), et en ce que des moyens libérables (28, 30, 32) sont destinés à fixer un rebord du couvercle à l'élément (2).

7. Dispositif et élément de recouvrement selon la revendication 6, caractérisés en ce que les moyens libérables comprennent une bride (28) partant d'une partie du rebord du couvercle (6) vers une partie opposée, et plusieurs ergots (32) faisant saillie de ladite face de l'élément (2), et plusieurs alvéoles correspondants (30) ménagés dans le couvercle, à proximité immédiate de son rebord, les ergots étant emboîtés sous pression dans les alvéoles.

8. Dispositif et couvercle selon la revendication 6, caractérisés en ce que l'élément (2) est traversé par au moins un trou (40) de ventilation au-dessous de ladite ouverture (12).

9. Dispositif et élément de recouvrement selon la revendication 5, caractérisés en ce que l'élément de recouvrement se présente sous la forme d'un sachet souple.

10. Dispositif selon la revendication 1, caractérisé en ce que l'élément est constitué d'une feuille d'épaisseur uniforme.

11. Modification du dispositif selon la revendication 1, caractérisé en ce qu'à la place dudit lobe, se trouve une longue patte avançant vers l'intérieur de l'ouverture, vers le haut et vers l'arrière de la partie antérieure inférieure du bord de l'ouverture, de manière à s'ajuster à la zone intertragienne et à correspondre à la forme de l'anthélix de l'auricule.

**Patentansprüche**

1. Ohrenschützer in Form eines selbsttragenden Plattengliedes (2) mit einer durch den mittigen Teil des Gliedes verlaufenden Öffnung (12), damit die Helix der Ohrmuschel hindurchgesteckt werden kann, wobei die hintere Begrenzung (16) der Öffnung nach hinten convex verläuft, dadurch gekennzeichnet, daß das Plattenglied im wesentlichen eben ist und daß die vordere Begrenzung an ihrer Mittelhöhe einen Lappen bzw. Zipfel (18) umfaßt, der nach hinten in die Öffnung hineinragt, so daß das Plattenglied auf der Ohrmuschel getragen wird, wobei der Lappen angrenzend am Tragus (20 = Knorpel vorn vor dem Gehörgang) anliegt.

2. Ohrenschützer nach Anspruch 1, dadurch gekennzeichnet, daß die Öffnung (12) einen Rand (34) aufweist, der abgerundeter und weicher ist als die Mehrheit des Plattengliedes (2).

3. Ohrenschützer nach Anspruch 1, dadurch gekennzeichnet, daß eine Fläche des Lappens (18) weicher ist als die Mehrheit des Plattengliedes (2).

4. Ohrenschützer nach Anspruch 1, dadurch gekennzeichnet, daß die Mehrheit des Plattengliedes (2) aus einem Silikon-Material besteht.

5. Ohrenschützer nach Anspruch 1, gekennzeichnet durch eine Kombination mit einer Abdeckung (6), die an einer Fläche des Gliedes (2) befestigt, oder befestigbar ist, um einen Raum (24) einzuschließen, der an die Fläche angrenzt.

6. Ohrenschützer und Abdeckung nach Anspruch 5, dadurch gekennzeichnet, daß die Abdeckung die Form einer selbsttragenden Kappe (6) besitzt, und daß wiederlösbare Mittel (28, 30, 32) zur Befestigung des Kappenrandes am Glied (2) vorgesehen sind.

7. Ohrenschützer und Abdeckung nach Anspruch 6, dadurch gekennzeichnet, daß die wiederlösbaren Mittel aus einem Flansch (28), der sich von einem Teil des Kappenrandes (6) hin zu einem gegenüberliegenden Teil erstreckt, sowie einer Vielzahl von Bolzen (32), die aus der genannten Fläche des Gliedes (2) hervorragen und einer entsprechenden Vielzahl an Fassungen (30) in der Kappe an seinem Rand bestehen, wobei die Bolzen als Schiebesitz in den Fassungen sitzen.

8. Ohrenschützer und Kappe nach Anspruch 6, dadurch gekennzeichnet, daß mindestens ein Belüftungsloch (40) im Glied (2) unterhalb der Öffnung (12) vorgesehen ist.

9. Ohrenschützer und Abdeckung nach Anspruch 5, dadurch gekennzeichnet, daß die Abdeckung die Form einer flexiblen Tasche besitzt.

10. Ohrenschützer nach Anspruch 1, dadurch gekennzeichnet, daß das Glied aus einem Plattenmaterial mit gleichmäßiger Dicke besteht.

11. Modifikation des Ohrenschützers nach Anspruch 1, dadurch gekennzeichnet, daß anstelle des Lappens (18) deine längliche Zunge vorgesehen ist, die sich in die Öffnung nach oben und hinten aus dem vorderen unteren Teil der Öffnungsbegrenzung erstreckt, um in den integralen Bereich zu passen und der Form der Antihelix der Ohrenmuschel zu entsprechen.

# 0 047 270

**Fig.1.**

**Fig.2.**

1

0 047 270

Fig.3.

Fig.4.

Fig.5.

14
2
32

20
14
22

VII
34
12
16
18
2
40
VII

2

Fig. 6.

Fig. 7.